# EUROPÄISCHE PATENTSCHRIFT

(11) **EP 2 056 724 B1**
(45) Veröffentlichungstag und Bekanntmachung des Hinweises auf die Patenterteilung: **28.07.2010**
(21) Anmeldenummer: 07786633.3
(22) Anmeldetag: 09.08.2007
(51) Int. Cl.: A61B 17/00

(54) **MEDIZINISCHE VORRICHTUNG**
MEDICAL DEVICE
DISPOSITIF MÉDICAL

(30) Priorität: 25.09.2006 DE 102006045545
(43) Veröffentlichungstag der Anmeldung: 13.05.2009
(73) Patentinhaber: Peter Osypka Stiftung Stiftung des bürgerlichen Rechts, 79639 Grenzach-Wyhlen (DE)
(72) Erfinder: OSYPKA, Peter, 79618 Rheinfelden-Herten (DE)
(74) Vertreter: Maucher, Wolfgang
(86) Internationale Anmeldenummer: PCT/EP2007/007043
(87) Internationale Veröffentlichungsnummer: WO 2008/037322

(56) Entgegenhaltungen:
- US-A- 5 725 552

## Beschreibung

Die Erfindung betrifft eine medizinische Vorrichtung, insbesondere ein Okklusionsinstrument, zum Verschließen angeborener oder durch Krankheiten gebildeter Öffnungen des menschlichen Körpers mit einem kollabierbaren Geflecht von Drähten und/oder Fäden aus Nitinol oder dergleichen Material mit Formengedächtnis oder Memoryeffekt, wobei die Enden der Drähte oder Fäden des Geflechts an mindestens einer Schließeinrichtung zusammengeführt und dort gehalten sind, wobei die Schließeinrichtung durch eine Hülse gebildet ist, in welche die Enden eingeführt und durch Klemmung gegen Entweichen aus der Hülse gehindert sind.

Derartige Vorrichtungen werden heutzutage häufig bei der Behandlung von Herzerkrankungen mittels Herzkathetertechnik eingesetzt und sind zum Beispiel aus der US 5,725,552, der US 5,846,261, der DE 103 38 702 B3 oder der DE 695 34 505 T2 bekannt. Mittels der Vorrichtungen können die auf unterschiedlichen Ursachen basierenden Öffnungen beispielsweise von Atrium-Septum-Defekten, etwa ein persistierendes Foramen ovale oder ein sogenannter Defekt vom Sekundumtyp, durch den Einsatz einer optimalerweise selbstzentrierenden Vorrichtung als Verschlusssystem, das nachträglich von Gewebe um- und überwachsen wird, zuverlässig behandelt werden. Eine solche medizinische Vorrichtung wird hierbei üblicherweise in einer Röhre kollabiert über eine Beinvene in den Körper eingebracht und mittels eines "Pusher" genannten Einführungskatheters in Richtung des Herzens geschoben. Nach Abziehen der Röhre entfaltet sich die Vorrichtung aufgrund des Formengedächtnisses ihres Materials in die ihr vorher zugewiesene Form und lässt sich an ihrem vorgesehenen Einsatzort in Position bringen und festlegen.

Bei den vorstehend erwähnten Vorrichtungen aus dem Stand der Technik lässt sich jedoch das Geflecht aus Nitinoldrähten oder -fäden häufig nur umständlich handhaben, da die freien Enden der Drähte durch eine Schließeinrichtung zusammengeführt und festgelegt werden müssen und gleichzeitig ein Möglichkeit geboten sein muss, einen Einführungskatheter an der Vorrichtung anzuschließen. Dies wird durch die Tatsache erschwert, dass sich Nitinol mit Stahl nicht verschweißen lässt. Ebenso ist an Nitinol nur sehr schwer durch Schneiden oder Materialabtrag beispielsweise ein Gewinde in den erforderlichen geringen Abmessungen als Anschluss formbar.

Die Festlegung der Drähte und Fäden an einer eine Klemmung bewirkenden Hülse hängt dabei davon ab, wie gut diese Klemmung bei der Montage herbeigeführt wird. Falls der Klemmvorgang nur unzureichend durchgeführt wird, hat die Vorrichtung einen entsprechenden Mangel.

Es besteht daher die Aufgabe, eine entsprechende medizinische Vorrichtung zu schaffen, die eine sichere Verbindung der freien Enden der Drähte des Geflechts gewährleistet und welche einfach handhabbar, schnell und zuverlässig mit einer Positioniereinrichtung lösbar verbindbar ist.

Diese Aufgabe wird erfindungsgemäß dadurch gelöst, dass die Schließeinrichtung mit einem hülsenfremden oder an der Hülse angeordneten Klemmelement versehen ist, das einen Kraft- und/oder Formschluss der Enden der Drähte oder Fäden des Geflechts mit der Hülse bewirkt, und dass das Klemmelement die Hülse durchgreift und an seinem dem Geflecht abgewandten Ende an einem Halteelement festgelegt ist.

Die erwähnte Hülse umfängt demnach die Drahtenden, die an irgendeiner Stelle ihres Durchgriffs der Hülse mit der benachbarten Innenwand derselben, insbesondere in deren Rand oder Stirnbereich einen Klemmsitz bilden, wodurch zum einen die Drahtenden an der Hülse zug- und/oder schubfest festgelegt sind, zum anderen an der Hülse, gegebenenfalls über ein weiteres Verbindungsmittel, eine Positioniereinrichtung für die medizinische Vorrichtung angeordnet werden kann. Die Hülse asl Schließeinrichtung und gegebenenfalls weitere an ihr anzuordnende Gegenstände können beispielsweise aus einem implantierbaren, biokompatiblen, Edelstahl bestehen.

Eine gute Klemmung zwischen der Hülse und den Drahtenden wird also dadurch erreicht, dass das Klemmelement zwischen die Drahtenden greift und sie derart auseinanderdrückt, dass eine gute klemmende Verbindung zwischen den Drahtenden und der Hülse zustande kommt, so dass die Klemmwirkung nicht durch ein Zusammendrücken der Hülse selbst bewirkt werden muss. Durch das Halteelement wird diese Klemmverbindung gesichert, da damit ein hülsenfremdes Klemmelement am Entweichen aus seinem Sitz innerhalb der Hülse gehindert werden kann.

Eine zweckmäßige und vorteilhafte Weiterbildung der erfindungsgemäßen medizinischen Vorrichtung kann darin bestehen, dass das Klemmelement der Schließeinrichtung durch zumindest einen wenigstens bereichsweise konischen Bolzen oder Stift gebildet ist, welcher zumindest teilweise in die Hülse eingreift. Dabei muss also nicht notwendigerweise der besagte Bolzen oder Stift als Klemmelement mit seiner gesamten Länge in die Hülse eingreifen, sondern kann über die Hülseöffnung ragen, wobei der konische Bereich des Klemmelements sich bevorzugt zumindest im Bereich der Eintrittsöffnung des Klemmelementes in die Hülse befindet und so dort, besonders am Rand der betreffenden Öffnung, für eine gute Klemmung der Enden des Geflechts sorgt.

Da sich das erwähnte Halteelement zweckmäßigerweise gut an die Form des Schließelements anpassen sollte, kann eine vorteilhafte Weiterbildung darin bestehen, dass das Halteelement scheibenförmig ausgebildet ist und vorzugsweise den gleichen Querschnitt wie die Hülse, insbesondere einen kreisrunden Querschnitt, aufweist. Hierdurch kann außerdem die den Enden der Geflechtsdrähte zugewandte Hülsenöffnung optimal geschlossen werden.

Eine zuverlässige Festlegung des Klemmelements an dem Halteelement erreicht man in einfacher Weise bei einer anderen Weiterbildung der Vorrichtung, bei der das Halteelement Durchtrittsöffnungen für das oder die Enden der Klemmelemente aufweist und diese Enden im Bereich des Ausgangs der Durchtrittsöffnungen dauerhaft mit dem Halteelement, insbesondere durch eine Schweißverbindung, verbunden sind. Hierbei können beispielsweise das oder die Enden der Klemmelemente die Durchtrittsöffnungen der Haltelemente vollständig durchgreifen und so bearbeitet werden, dass sie den Rand des Ausgangs der jeweiligen Durchtrittsöffnung übergreifen und schon hierdurch an einer entgegengesetzten Bewegung aus der Öffnung hinaus gehindert sind und dann zum Beispiel noch zusätzlich verschweißt werden.

Zur Verhinderung eines möglicherweise schädlichen Einflusses offenliegender Drahtenden des Geflechts an der Austrittsöffnung der Hülse kann mit einer anderen Weiterbildung der medizinischen Vorrichtung begegnet werden, bei der das Halteelement an seiner dem Geflecht zugewandten Flachseite mit dem benachbarten Stirnende der Hülse verbunden ist. Im Ergebnis entsteht hierdurch eine nur noch zu dem Geflecht hin offene Hülse. Die erwähnte Verbindung zwischen Halteelement und Hülse Verbindung lässt sich besonders einfach erneut durch eine Schweißverbindung realisieren.

Eine andere Möglichkeit, die Klemmverbindung zwischen Drahtenden und Hülse in einfacher Art und Weise zu verwirklichen, stellt eine Ausführungsform der Erfindung dar, bei der die Hülse an ihren Enden einen unterschiedlichen Öffnungsquerschnitt aufweist und die Öffnung zwischen den Enden konisch verläuft. Die Klemmung wird bei dieser Ausführungsform bevorzugt an der Hülsenöffnung mit dem kleineren Öffnungsquerschnitt gebildet, welche wiederum bevorzugt dem Draht- oder Fadengeflecht zugewandt ist. Die Klemmung kann dabei zusätzlich von einem Klemmelement unterstützt werden.

In besonders gut handzuhabender Weise gelingt die Unterstützung mittels des Klemmelements bei einer Weiterbildung der medizinischen Vorrichtung, bei der das Klemmelement von dem Hülsenende mit größerem Öffnungsquerschnitt in die Hülse eingeführt und gegebenenfalls mit dem Halteelement einstückig ausgebildet ist. Das Klemmelement kann hierbei beispielsweise über seine gesamte Länge konisch und in Richtung auf das Drahtgeflecht hin spitz zulaufend ausgebildet sein, wobei in einstückiger Ausführung mit dem Halteelement diese lediglich noch an der Hülse festgelegt werden muss.

Ein von den Abmessungen der Hülse als Schließeinrichtung des Drahtgeflechts unabhängiger Anschluss der medizinischen Vorrichtung weitere Einrichtungen, insbesondere eine Positioniereinrichtung, an welcher die medizinische Vorrichtung während einer Einführphase in den Körper anzuschließen ist, kann bei einer gegebenenfalls eigenständig erfinderischen Ausführungsform der medizinischen Vorrichtung verwirklicht werden, bei der an der Hülse ein insbesondere hülsenförmiges Verbindungsstück anordenbar ist, welches die Hülse zumindest teilweise übergreift. Dieses Verbindungsstück, in welches die Hülse, wenn ihr Außendurchmesser in etwa dem Innendurchmesser der Öffnung des Verbindungsstücks entspricht, ungefähr bündig zumindest teilweise eingreift, bildet eine Art Adapter zwischen der Schließeinrichtung und der erwähnten, meist als Einführkatheter ausgebildeten Positioniereinrichtung.

Es kann dabei vorteilhafterweise in einer Weiterbildung eine oder mehrere Anschlusseinrichtungen verschiedener Art aufweisen, von denen eine besonders bevorzugte durch ein an dem Verbindungsstück angeordnetes Gewinde, beispielsweise ein M1-Gewinde, gebildet ist. Es sind aber auch andere Anschlusseinrichtungen vorstellbar, ebenso wie ein vollständiger Verzicht auf eine Verbindungshülse, wobei dann die Schließeinrichtung mit einer Anschlusseinrichtung, beispielsweise einem Außengewinde, zu versehen wäre. In seiner Ausbildung als hülsenförmiges Verbindungsstück ist dieses mit der Hülse des Schließelements besonders einfach formschlüssig dauerhaft zu verbinden, erneut zum Beispiel durch eine Schweißverbindung. Die Zuverlässigkeit der Verbindung wird noch erhöht, wenn der überwiegende Teil der Hülse in der Öffnung des Verbindungsstücks aufgenommen ist.

Eine vorteilhafte Maßnahme, um etwa ein zwischen den in der Hülse befindlichen Drahtenden befindliches Klemmelement gegen mechanische Einflüsse zu schützen kann bei einer anderen Ausführung der Erfindung darin bestehen, dass zwischen der Hülse und dem Drahtgeflecht eine vorzugsweise als Klammer ausgebildete Schutzeinrichtung angeordnet ist, deren Enden die Hülse und gegebenenfalls das Verbindungsstück zumindest teilweise übergreifen. Die Festlegung der Schutzeinrichtung richtete sich danach, welches der erwähnten Teile zuäußerst übergriffen ist.

Wie bereits in der vorstehenden Beschreibung angedeutet, lassen sich insbesondere dauerhafte Verbindungen zwischen den einzelnen Teilen besonders gut durch Schweißverbindungen, insbesondere Laserschweißverbindungen herstellen, was wiederum schweißbare Materialien voraussetzt. Daher sind zweckmäßigerweise bei einer Ausführung der medizinischen Vorrichtung die Hülse und/oder das Klemmelement und/oder das Halteelement und/oder das Verbindungsstück aus einem schweißbaren implantierbaren Material, insbesondere einem Edelstahl, ausgebildet. Ebenso ist es aber bei einer anderen Ausführung der Vorrichtung denkbar, dass die Hülse aus Nitinol gebildet ist und dann beispielsweise das die eine Hülsenöffnung verschließende Halteelement mit dem Verbindungsstück dauerhaft über eine Schweißverbindung verbunden wird.

In seiner Handhabung besonders einfach, weil gut kollabierbar und wieder entfaltbar ist eine Ausführung der medizinischen Vorrichtung, bei der die Schließeinrichtung im Falle eines zylindersymmetrischen Geflechts koaxial zu dessen Hauptachse angeordnet ist.

Die Erfindung wird nachstehend anhand von Ausführungsbeispielen in der Zeichnung näher erläutert. Dabei zeigen in zum Teil schematisierter Darstellung die
- Fig. 1: eine perspektivische Seitenansicht einer medizi- nischen Vorrichtung in Gebrauchsstellung mit ei- ner an deren oberen Ende angeordneten Schließ- einrichtung,
- Fig. 2: eine geschnittene Seitenansicht einer ersten Ausführungsform der Schließeinrichtung mit einem Klemmelement und einem Verbindungsstück mit An- schlusseinrichtung,
- Fig. 3: die Schließeinrichtung der Fig.2 mit daran ange- ordneter Schutzeinrichtung und die
- Fig. 4: eine geschnittene Seitenansicht einer anderen Ausführungsform der Schließeinrichtung, bei der die Hülse konisch und das Klemmelement mit dem Halteelement einstückig ausgebildet ist.

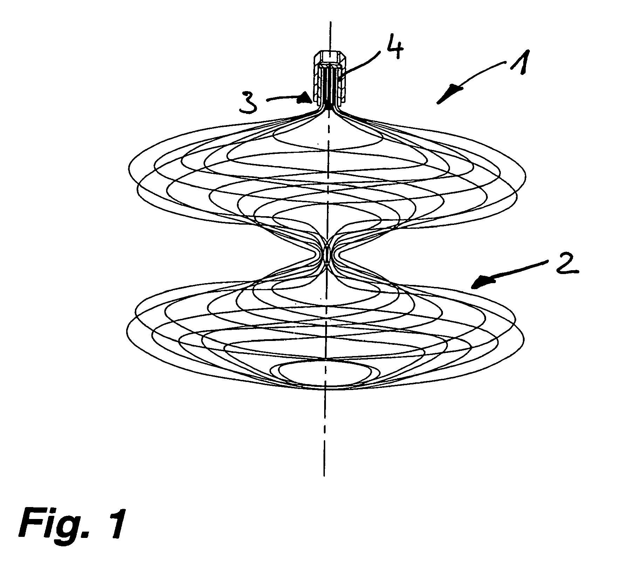

In der Fig.1 ist in Gebrauchsstellung eine im Ganzen mit 1 bezeichnete medizinische Vorrichtung, ein sogenannter ASD-Verschluss, zu erkennen, die zum Verschließen angeborener oder durch Krankheiten gebildeter Öffnungen des menschlichen Körpers verwendet werden kann. Die medizinische Vorrichtung 1 weist ein kollabierbares Geflecht 2 von Drähten und/oder Fäden aus Nitinol oder dergleichen Material mit Formengedächtnis oder Memoryeffekt auf, so dass das Geflecht 2 mit gestreckten Drähten in einem nicht dargestellten Katheter in den menschlichen Körper eingebracht werden kann, wobei die Enden 3 der Drähte oder Fäden des Geflechts an einer Schließeinrichtung 4 zusammengeführt und dort gehalten sind.

Die Schließeinrichtung 4 ist durch eine Hülse, hier eine Edelstahlhülse, gebildet, in welche die Enden 3 eingeführt und durch Klemmung gegen Entweichen aus der Hülse gehindert sind. Die an dem für den Betrachter oberen Ende der medizinischen Vorrichtung angeordnete Schließeinrichtung 4 weist an ihrem dem Geflecht 2 abgewandten Ende ein Gewinde als Anschlusseinrichtung 9 auf, mittels dessen eine ebenfalls nicht dargestellte Positioniereinrichtung, beispielsweise ein Einführkatheter, an der Schließeinrichtung 4 und damit dem beschriebenen Verbund angeordnet werden und dieser dadurch bewegt und dirigiert werden kann. Nach erfolgter Einführung der Vorrichtung 1, z.B. in einen Vorhof des Herzens, und sich aufgrund des Formengedächtnisse ihres Materials anschließender Entfaltung in die in der Fig.1 gezeigte Gebrauchsstellung kann der betreffende Einführkatheter abgezogen werden

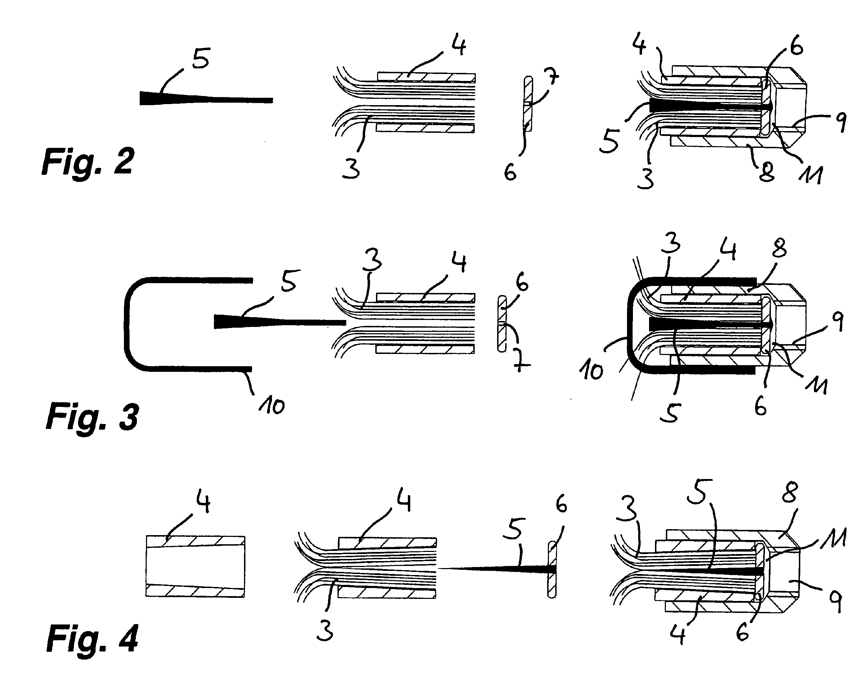

In der Fig.2 ist in höherer Detailgenauigkeit eine erste Ausführungsform der durch eine Hülse gebildeten Schließeinrichtung 4 zu erkennen, die mit einem hülsenfremden Klemmelement 5 in Form eines Bolzens aus Edelstahl versehen ist. In der Fig.2 ist zunächst links der Bolzen, sodann rechts davon die Schließeinrichtungr 4 mit darin eingeführten Enden 3 von Drähten und wiederum rechts von dieser ein scheibenförmiges Halteelement 6 aus Edelstahl zu erkennen. Ganz rechts in der Fig.2 ist die Schließeinrichtung in zusammengefügtem Zustand mit daran angeordnetem Verbindungsstück 8 gezeigt.

Weiter ist der Fig.2 zu entnehmen, dass also das Klemmelement 5 von links zwischen die aus der Hülse hervortretenden, und in das Geflecht übergehenden Enden 3 von Drähten in die Hülsenöffnung eingeführt wird. Der Bolzen ist bereichsweise konisch ausgebildet und greift in dem rechts dargestellten zusammengefügten Zustand mit dem überwiegenden Teil seiner Länge in die Hülse ein, bzw. durchgreift sie. Im konischen Bereich des Bolzens werden die Drähte im Zusammenwirken mit der Innenwand und dem Rand der Hülse dabei besonders stark geklemmt. Das scheibenförmige Halteelement 6 dient zur Festlegung des durch die Hülse greifenden Endes des Klemmelements 5. Hierzu ist das Haltelement 6, das im übrigen im wesentlichen den gleichen Querschnitt wie die Hülse aufweist, selbst mit einer von dem Ende des Klemmelements 5 durchgriffenen Durchtrittsöffnung 7 versehen und das Ende des Klemmelements ist im dem Drahtgeflecht 3 abgewandten Ausgangsbereich der Durchtrittsöffnung 7 mit diesem Halteelement 6 verschweißt, so dass das Klemmelement 5 nicht in Richtung nach links aus der Hülse entweichen kann. Das Halteelement 6 wiederum ist an seiner dem Drahtgeflecht 3 zugewandten Flachseite mit dem benachbarten Stirnende der Hülse dauerhaft, nämlich erneut durch eine Schweißverbindung, verbunden.

Im zusammengefügten Zustand ist an der Schließeinrichtung 4 ein hülsenförmiges Verbindungsstück 8 angeordnet, welches mit einer Öffnung 11 versehen ist, in welche die Schließeinrichtung 4 eingreift, so dass das Verbindungsstück 8 die Hülse der Schließeinrichtung 4 teilweise übergreift. Das Verbindungsstück 8 ist hierbei mit der Hülse an Berührungsstellen dauerhaft mittels einer Schweißverbindung verbunden und weist nach rechts gerichtet eine Anschlusseinrichtung 9 in Art eines Gewindes zur Anordnung einer nicht dargestellten Positioniereinrichtung für die Vorrichtung 1 auf.

Der in der Fig.3 dargestellte Sachverhalt entspricht demjenigen aus der Fig.2 mit der Ausnahme, dass zwischen der Hülse und dem Drahtgeflecht 2 eine als Klammer ausgebildete Schutzeinrichtung 10 angeordnet ist, deren Enden die Hülse und gegebenenfalls das Verbindungsstück 8 teilweise übergreifen. Die Innenseiten der Klammerenden kommen dabei auf den Außenseiten des Verbindungsstücks 8 zu liegen und sind erneut dauerhaft mit dieser verbunden, so dass die Klammer insgesamt den Bolzen gegen mechanische Einflüsse schützen kann, während die in das Geflecht 2 übergehenden Enden 3 der Drähte seitlich an der Klammer vorbeigeführt sind.

In der Fig.4 ist eine andere Ausführungsform der Schließeinrichtung 4 in Einzelteilen und im zusammengefügtem Zustand gezeigt, bei der die ganz links dargestellte Hülse an ihren Enden einen unterschiedlichen Öffnungsquerschnitt aufweist und die Öffnung zwischen den Enden konisch verläuft. Bei Einführung der Enden 3 der Drähte in die Hülse in der Mitte der Darstellung sind diese an dem engeren Austrittsende der Enden 3 schon in gewisser Wiese beengt. Daraufhin wird das einstückig mit dem Halteelement 6 verbundene Klemmelement 5 von dem Hülsenende mit größerem Öffnungsquerschnitt in die Hülse eingeführt und das Halteelement erneut in bereits bekannter Weise mit dem Stirnende der Hülse verbunden, Auch die Aufnahme und Verbindung mit dem Verbindungsstück ist bereits aus den Fign. 2 und 3 bekannt.

Demnach betrifft die vorstehend beschriebene Erfindung also eine medizinische Vorrichtung 1, insbesondere Okklusions-instrument, zum Verschließen angeborener oder durch Krankheiten gebildeter Öffnungen des menschlichen Körpers mit einem kollabierbaren Geflecht 2 von Drähten und/oder Fäden aus Nitinol oder dergleichen Material mit Formengedächtnis oder Memoryeffekt, wobei die Enden 3 der Drähte oder Fäden des Geflechts an einer Schließeinrichtung 4 zusammengeführt und dort gehalten sind. Die Vorrichtung wird dadurch besonders gut handhabbar und mit Anschlusseinrichtungen verbindbar, dass die Schließeinrichtung 4 durch eine Hülse gebildet ist, in welche die Enden 3 eingeführt und durch Klemmung gegen Entweichen aus der Hülse gehindert sind.

Eine gute Klemmung bewirkt dabei ein hülsenfremdes Klemmelement 5.

## Patentansprüche

1. Medizinische Vorrichtung (1), insbesondere Okklusionsinstrument, zum Verschließen angeborener oder durch Krankheiten gebildeter Öffnungen des menschlichen Körpers mit einem kollabierbaren Geflecht (2) von Drähten und/oder Fäden aus Nitinol oder dergleichen Material mit Formengedächtnis oder Memoryeffekt, wobei die Enden (3) der Drähte oder Fäden des Geflechts an mindestens einer Schließeinrichtung (4) zusammengeführt und dort gehalten sind, wobei die Schließeinrichtung (4) durch eine Hülse gebildet ist, in welche die Enden (3) eingeführt und durch Klemmung gegen Entweichen aus der Hülse gehindert sind, **dadurch gekennzeichnet, dass** die Schließeinrichtung (4) mit einem hülsenfremden oder an der Hülse angeordneten Klemmelement (5) versehen ist, das einen Kraft- und/oder Formschluss der Enden (3) der Drähte oder Fäden des Geflechts (2) mit der Hülse bewirkt und dass das Klemmelement (5) die Hülse durchgreift und an seinem dem Geflecht (2) abgewandten Ende an einem Halteelement (6) festgelegt ist.

2. Medizinische Vorrichtung nach Anspruch 1, **dadurch gekennzeichnet, dass** das Klemmelement (5) der Schließeinrichtung (4) durch zumindest einen wenigstens bereichsweise konischen Bolzen oder Stift gebildet ist, welcher zumindest teilweise in die Hülse eingreift.

3. Medizinische Vorrichtung nach Anspruch 1 oder 2, **dadurch gekennzeichnet, dass** das Halteelement (6) scheibenförmig ausgebildet ist und vorzugsweise den gleichen Querschnitt wie die Hülse, insbesondere einen kreisrunden Querschnitt, aufweist.

4. Medizinische Vorrichtung nach einem der Ansprüche 1 bis 3, **dadurch gekennzeichnet, dass** das Halteelement (6) Durchtrittsöffnungen (7) für das oder die Enden der Klemmelemente (5) aufweist und diese Enden im Bereich des Ausgangs der Durchtrittsöffnungen (7) dauerhaft mit dem Halteelement (6), insbesondere durch eine Schweißverbindung, verbunden sind.

5. Medizinische Vorrichtung nach einem der Ansprüche 1 bis 4, **dadurch gekennzeichnet, dass** das Halteelement (6) an seiner dem Geflecht (2) zugewandten Flachseite mit dem benachbarten Stirnende der Hülse verbunden ist.

6. Medizinische Vorrichtung nach einem der vorhergehenden Ansprüche, **dadurch gekennzeichnet, dass** die Hülse an ihren Enden einen unterschiedlichen Öffnungsquerschnitt aufweist und die Öffnung zwischen den Enden konisch verläuft.

7. Medizinische Vorrichtung nach einem der Ansprüche 1 bis 6, **dadurch gekennzeichnet, dass** das Klemmelement (5) von dem Hülsenende mit größerem Öffnungsquerschnitt in die Hülse eingeführt und gegebenenfalls mit dem Halteelement einstückig ausgebildet ist.

8. Medizinische Vorrichtung nach einem der vorhergehenden Ansprüche, **dadurch gekennzeichnet, dass** an der Schließeinrichtung (4) ein insbesondere hülsenförmiges Verbindungsstück (8) anordenbar ist, welches die Schließeinrichtung (4) zumindest teilweise übergreift.

9. Medizinische Vorrichtung nach Anspruch 8, **dadurch gekennzeichnet, dass** das Verbindungsstück (8) mit der Hülse dauerhaft, insbesondere mittels einer Schweißverbindung verbindbar ist.

10. Medizinische Vorrichtung nach Anspruch 8 oder 9, **dadurch gekennzeichnet, dass** an dem Verbindungsstück (8) mindestens eine Anschlusseinrichtung (9), insbesondere in Art eines Gewindes, zur Anordnung einer Positioniereinrichtung für die Vorrichtung (1) vorgesehen ist.

11. Medizinische Vorrichtung nach einem der vorhergehenden Ansprüche, **dadurch gekennzeichnet, dass** zwischen der Hülse und dem Drahtgeflecht (2) eine vorzugsweise als Klammer ausgebildete Schutzeinrichtung (10) angeordnet ist, deren Enden die Hülse und gegebenenfalls das Verbindungsstück (8) zumindest teilweise übergreifen.

12. Medizinische Vorrichtung nach einem der vorhergehenden Ansprüche, **dadurch gekennzeichnet, dass** die Hülse und/oder das Klemmelement (5) und/oder das Halteelement (6) und/oder das Verbindungsstück (8) aus einem schweißbaren implantierbaren Material, insbesondere einem Edelstahl, ausgebildet sind.

13. Medizinische Vorrichtung nach einem der vorhergehenden Ansprüche, **dadurch gekennzeichnet, dass** die Hülse aus Nitinolmaterial besteht.

14. Medizinische Vorrichtung nach einem der vorhergehenden Ansprüche, **dadurch gekennzeichnet, dass** die Schließeinrichtung (4) im Falle eines zylindersymmetrischen Geflechts (2) koaxial zu dessen Hauptachse angeordnet ist.

## Claims

1. Medical device (1), particularly an occlusion instrument, for closing up congenital or pathologically caused openings in the human body, with a collapsible meshwork (2) of wires and/or filaments of nitinol or similar material with shape memory or memory effect, wherein the ends (3) of the wires or filaments of the meshwork are brought together at at least one closure device (4) and held In place there, the closure device (4) being formed by a sleeve into which the ends (3) are Inserted and are prevented from escaping from the sleeve by damping, **characterised in that** the closure device (4) is provided with a clamping element (5) extraneous to the sleeve or mounted on the sleeve, which produces a frictional and/or Interlocking engagement of the ends (3) of the wires or filaments of the meshwork (2) with the sleeve, and **in that** the clamping element (5) passes through the sleeve and is secured to a retaining element (6) at its end remote from the meshwork (2).

2. Medical device according to claim 1, **characterised in that** the clamping element (5) of the closure device (4) Is formed by at least one bolt or pin that is conical at least In parts and that engages at least partly in the sleeve.

3. Medical device according to claim 1 or 2, **characterised in that** the retaining element (6) is of disc-shaped construction and preferably has the same cross-section as the sleeve, particularly a circular cross-section.

4. Medical device according to one of claims 1 to 3, **characterised in that** the retaining element (6) has through-openings (7) for the end or ends of the clamping elements (5) and these ends are durably connected to the retaining element (6) In the region of the exit from the through-openings (7), particularly by means of a weld Joint.

5. Medical device according to one of claims 1 to 4, **characterised in that** the retaining element (6) is connected, by its flat side facing the meshwork (2), to the adjacent end face of the sleeve.

6. Medical device according to one of the preceding claims, **characterised in that** the sleeve has a different cross-section of opening at its ends and the opening extends conically between the ends.

7. Medical device according to one of claims 1 to 6, **characterised in that** the clamping element (5) is inserted into the sleeve from the sleeve end with the larger cross-section of opening and is optionally formed in one piece with the retaining element.

8. Medical device according to one of the preceding claims, **characterised in that** an In particular sleeve-shaped connecting member (8) can be arranged on the closure device (4), said connecting member (8) at least partly overlapping the closure device (4).

9. Medical device according to claim 8, **characterised in that** the connecting member (8) is durably connectable to the sleeve, particularly by means of a weld joint.

10. Medical device according to claim 8 or 9, **characterised in that** at least one attachment device (9) is provided on the connecting member (8), particularly in the manner of a screw thread, for the purpose of mounting a positioning device for the device (1).

11. Medical device according to one of the preceding claims, **characterised in that** between the sleeve and, the wire meshwork (2) is mounted a protective device (10), preferably in the form of a clip, the ends of which at least partially overlap the sleeve and optionally the connecting member (8).

12. Medical device according to one of the preceding claims, **characterised in that** the sleeve and/or the clamping element (5) and/or the retaining element (6) and/or the connecting member (8) is or are made from a weldable implantable material, particularly a stainless steel.

13. Medical device according to one of the preceding claims, **characterised in that** the sleeve consists of nitinol material.

14. Medical device according to one of the preceding claims, **characterised in that** in the case of a cylindrically-symmetrlcal meshwork (2) the closure device (4) is arranged coaxially with the main axis thereof.

## Revendications

1. Dispositif médical (1), notamment instrument d'occlusion conçu pour obturer des orifices congénitaux ou induits par des maladies et présents dans le corps humain, muni d'une structure tressée (2) apte à l'écrasement, constituée par des fils métalliques et/ou des fils en Nitinol ou en un matériau similaire à mémoire de forme ou à effet mémoire, les extrémités (3) desdits fils métalliques ou fils de ladite structure tressée étant regroupées au niveau d'au moins un système de fermeture (4), par lequel elles sont retenues, ledit système de fermeture (4) étant formé d'une douille dans laquelle lesdites extrémités (3) sont insérées et empêchées, par coincement, de s'extirper de ladite douille, **caractérisé par le fait que** ledit système de fermeture (4) est pourvu d'un élément de coincement (5) distinct de la douille ou placé sur ladite douille, et provoquant une coopération par complémentarité de forces, et/ou par concordance de formes, entre ladite douille et lesdites extrémités (3) des fils métalliques ou fils de ladite structure tressée (2) ; et **par le fait que** ledit élément de coincement (5) traverse ladite douille et est consigné à demeure, sur un élément de retenue (6), à son extrémité tournée à l'opposé de ladite structure tressée (2).

2. Dispositif médical selon la revendication 1, **caractérisé par le fait que** l'élément de coincement (5) du système de fermeture (4) est constitué d'au moins un tenon ou une cheville de configuration tronconique, au moins par zones, qui pénètre au moins partiellement dans la douille.

3. Dispositif médical selon la revendication 1 ou 2, **caractérisé par le fait que** l'élément de retenue (6) est de réalisation discoïdale et offre, de préférence, la même section transversale que la douille, en particulier une section transversale circulaire.

4. Dispositif médical selon l'une des revendications 1 à 3, **caractérisé par le fait que** l'élément de retenue (6) présente des ouvertures de passage (7) dédiées à l'extrémité ou aux extrémités des éléments de coincement (5), et ces extrémités sont reliées durablement audit élément de retenue (6), notamment par une jonction soudée, dans la région de la sortie desdites ouvertures de passage (7).

5. Dispositif médical selon l'une des revendications 1 à 4, **caractérisé par le fait que** l'élément de retenue (6) est relié, par sa face aplatie tournée vers la structure tressée (2), à l'extrémité frontale de la douille qui occupe une position voisine.

6. Dispositif médical selon l'une des revendications précédentes, **caractérisé par le fait que** la douille possède une section transversale d'ouverture différente, à ses extrémités, et ladite ouverture s'étend tronconiquement entre lesdites extrémités.

7. Dispositif médical selon l'une des revendications 1 à 6, **caractérisé par le fait que** l'élément de coincement (5) est introduit dans la douille à partir de l'extrémité de ladite douille dont la section transversale d'ouverture est supérieure ; et est, le cas échéant, réalisé d'un seul tenant avec l'élément de retenue.

8. Dispositif médical selon l'une des revendications précédentes, **caractérisé par le fait qu'**une pièce de liaison (8), revêtant notamment la forme d'un capuchon et pouvant être mise en place sur le système de fermeture (4), coiffe au moins partiellement ledit système de fermeture (4).

9. Dispositif médical selon la revendication 8, **caractérisé par le fait que** la pièce de liaison (8) peut être reliée à la douille de manière durable, en particulier au moyen d'une jonction soudée.

10. Dispositif médical selon la revendication 8 ou 9, **caractérisé par le fait qu'**au moins un système de raccordement (9), notamment du type filetage, est prévu sur la pièce de liaison (8) en vue de la mise en place d'un système de positionnement dévolu audit dispositif (1).

11. Dispositif médical selon l'une des revendications précédentes, **caractérisé par le fait qu'**un système de protection (10) réalisé, de préférence, sous la forme d'une agrafe et dont les extrémités coiffent au moins partiellement la douille et, le cas échéant, la pièce de liaison (8), est interposé entre ladite douille et la structure (2) tressée en des fils métalliques.

12. Dispositif médical selon l'une des revendications précédentes, **caractérisé par le fait que** la douille et/ou l'élément de coincement (5) et/ou l'élément de retenue (6) et/ou la pièce de liaison (8) consiste(nt) en un matériau implantable apte au soudage, notamment en un acier fin.

13. Dispositif médical selon l'une des revendications précédentes, **caractérisé par le fait que** la douille est constituée d'un matériau de type Nitinol.

14. Dispositif médical selon l'une des revendications précédentes, **caractérisé par le fait que**, en présence d'une structure tressée (2) à symétrie cylindrique, le système de fermeture (4) est agencé coaxialement à l'axe principal de ladite structure.
